# EUROPEAN PATENT APPLICATION

(11) **EP 4 763 954 A1**
(43) Date of publication of application: **24.06.2026**
(21) Application number: 24222164.6
(22) Date of filing: 20.12.2024
(51) Int. Cl.: C10M 105/32, C09K 5/10, H01B 3/20, C10N 20/02, C10N 20/00, C10N 30/02, C10N 40/14, C10N 40/16

(54) **LOW POUR POINT FATTY ESTER COMBINATION**

(71) Applicant: OLEON NV, 9940 Evergem (Ertvelde) (BE)
(72) Inventor: LESCOFFIT, Anne-Elise, 60280 MARGNY-LES-COMPIEGNE (FR); KERBRAT, Marion, 60280 MARGNY-LES-COMPIEGNE (FR)
(74) Representative: Santarelli

(57) **Abstract**

The present invention relates to a specific combination of esters, a composition comprising it, and its uses as a lubricant and/or liquid dielectric coolant.

## Description

The present invention relates to a specific combination of esters, a composition comprising it and its uses as a lubricant and/or liquid dielectric coolant.

The specific combination of esters presents an unexpected low pour point. Indeed, usually when two chemicals are mixed, the pour point of the mixture is comprised between the pour point of each separate chemical. In the present invention, the pour point of the combination of esters is lower than the lowest pour point of the two esters.

The pour point of a fluid is the lowest temperature at which the fluid flows. Also, this characteristic is critical for applications in very cold environments.

For instance, for outdoor applications, lubricants still need to be liquid and pumpable at low temperature, such as at a temperature of at least -30°C. For applications in extreme environments, lubricants still need to be liquid and pumpable at even lower temperatures, such as at a temperature of at least -45°C.

A lubricant typically comprises a base oil, usually the major constituent (the constituent whose content is the highest), and one or more additive(s). An additive is used to enhance one or more intrinsic property(ies) of the base oil and / or to provide it with one or more additional property(ies).

Since base oils do not necessarily have a sufficiently low pour point, a pour point depressant is added as additive to base oils to lower the pour point.

Usual pour point depressants are polymers, such as polyalkylmethacrylates, polymethacrylates, polyacrylates, acrylate-styrene copolymers, esterified olefin- or styrene maleic anhydride copolymers, alkylated polystyrenes, and vinyl acetate-fumarate copolymers.

Those pour point depressants are not biodegradable and not obtained from renewable chemicals. Additionally, they are often developed for mineral base oils and have a limited impact on ester base oils, which are yet more and more developed as sustainable solutions.

Also, there is still a need for a sustainable ester base oil that would have a pour point lower than -45°C.

The Applicant surprisingly found that a specific combination of esters presents an improved pour point.

Accordingly, the present invention relates to a combination consisting of:
- 2-hexyl-1-decyl heptanoate; and
- propylene glycol dicaprylate/dicaprate;
wherein the weight ratio 2-hexyl-1-decyl heptanoate / propylene glycol dicaprylate/dicaprate is of about 70/30.

In the present application, unless otherwise indicated, all ranges of values used are to be understood as being inclusive limits.

In the present patent application, "about" is intended to mean more or less 2%, preferably more or less 1.5%, more preferably more or less 1%, even more preferably more or less 0.5%.

The combination according to the invention consists of about 70% by weight of 2-hexyl-1-decyl heptanoate and about 30% by weight of propylene glycol dicaprylate/dicaprate, weight percentages being based on the weight of the combination.

This specific combination presents an unexpected low pour point. The latter is indeed lower than the pour point of the chemicals present in the combination.

The pour point of the combination is lower by 7°C relative to the lowest pour point of esters contained in the combination, e.g. the pour point of 2-hexyl-1-decyl heptanoate. As illustrated in Example 2, the combination according to the invention has a pour point of - 64°C.

In the present application, the pour point is measured according to the standard ASTM D97.

Moreover, both 2-hexyl-1-decyl heptanoate and propylene glycol dicaprylate/ dicaprate may be obtained from renewable chemicals.

The combination according to the invention may be included in a composition further comprising at least an additive.

Thus, the invention also relates to a composition comprising:
- 2-hexyl-1-decyl heptanoate;
- propylene glycol dicaprylate/dicaprate; and
- at least an additive;
wherein the weight ratio 2-hexyl-1-decyl heptanoate / propylene glycol dicaprylate/dicaprate is of about 70/30.

Preferably, the additive is an additive usually used in the field of lubricants.

The additive(s) used in the field of lubricants, may be easily selected by a person skilled in the art who knows how to select the most suitable additive(s) and its/their quantity(ies) depending on the application. By way of example, reference may be made to the following manuals: "Fuels and Lubricants Handbook: technology, properties performance and testing", by George E. Totten, 2003 and "Handbook of lubrification and tribology, vol II : Theory and Design", by Robert W. Bruce, 2012.

The at least an additive used in the field of lubricants is preferably selected from the group consisting of antioxidants, corrosion inhibitors, metal deactivators, extreme pressure agents, anti-wear agents, friction modifiers, antifoam agents, dispersants, and mixtures thereof.

Preferably, the antioxidant is selected from the group consisting of phenolic compounds, amines, thiadiazoles, dialkyl dithiophosphates, amine phosphates, and mixtures thereof.

Preferably, the total quantity of antioxidant(s) represents at least 0.01%, more preferably at least 0.05%, even more preferably at least 0.1% by weight based on the weight of the composition.

Preferably, the total quantity of antioxidant(s) represents at most 5%, more preferably at most 3%, even more preferably at most 1.5% by weight based on the weight of the composition.

Preferably, the corrosion inhibitor is selected from the group consisting of alkali metal and/or alkaline earth metal sulphonates (Na, Mg, Ca salts), fatty acids, fatty amines, alkenylsuccinic acids and/or their derivatives, benzotriazole, tolyltriazole, and mixtures thereof.

Preferably, the total quantity of corrosion inhibitor(s) represents at least 0.01%, more preferably at least 0.05%, even more preferably at least 0.1% by weight based on the weight of the composition.

Preferably, the total quantity of corrosion inhibitor(s) represents at most 5 %, more preferably at most 3%, even more preferably at most 1.5% by weight based on the weight of the composition.

Preferably, the metal deactivator is selected from the group consisting of heterocyclic compounds containing nitrogen and/or sulphur, such as triazole, tolyltriazole, benzotriazole, and mixtures thereof.

Preferably, the total quantity of metal deactivator(s) represents at least 0.001%, more preferably at least 0.01% by weight based on the weight of the composition.

Preferably, the total quantity of metal deactivator(s) represents at most 0.050%, more preferably at most 0.025% by weight based on the weight of the composition.

Preferably, the extreme pressure agent is selected from the group consisting of compounds based on sulphur, phosphorus and/or chlorine, and mixtures thereof.

Preferably, the total quantity of extreme pressure agent(s) represents at least 0.01% by weight based on the weight of the composition.

Preferably, the total quantity of extreme pressure agent(s) represents at most 5 %, more preferably at most 1.5%, even more preferably at most 0.2% by weight based on the weight of the composition.

Preferably, the anti-wear agent is selected from the group consisting of polysulfides, thiocarbamates, chlorinated paraffins, phosphates and thio-phosphates, and mixtures thereof.

Preferably, the total quantity of anti-wear agent(s) represents at least 0.01%, more preferably at least 0.1%, even more preferably at least 0.2% by weight based on the weight of the composition.

Preferably, the total quantity of anti-wear agent(s) represents at most 5%, more preferably at most 4% by weight based on the weight of the composition.

Preferably, the friction modifier is selected from the group consisting of partial esters of fatty acids and polyhydroxy alcohols such as glycerol mono oleate (GMO), fatty amides such as oleate diethanolamide (ODEA), and mixtures thereof.

Preferably, the total quantity of friction modifier(s) represents at least 0.01%, more preferably at least 0.05%, even more preferably at least 0.1% by weight based on the weight of the composition.

Preferably, the total quantity of friction modifier(s) represents at most 5%, more preferably at most 2.5%, even more preferably at most 1% by weight based on the weight of the composition.

Preferably, the antifoam agent is selected from the group consisting of silicone oils, silicone polymers, alkyl acrylates, and mixtures thereof.

Preferably, the total quantity of antifoam agent(s) represents at least 0.01%, more preferably at least 0.1%, even more preferably at least 0.5% by weight based on the weight of the composition.

Preferably, the total quantity of antifoam agent(s) represents at most 5%, more preferably at most 3%, even more preferably at most 1.5% by weight based on the weight of the composition.

Preferably, the dispersant is selected from the group consisting of alkenylsuccinimides, succinic esters and derivatives thereof, Mannich bases, and mixtures thereof.

Preferably, the total quantity of dispersant(s) represents at least 0.01%, more preferably at least 0.1%, even more preferably at least 0.5% by weight based on the weight of the composition.

Preferably, the total quantity of dispersant(s) represents at most 15%, more preferably at most 10%, even more preferably at most 5% by weight based on the weight of the composition.

By "total quantity of X(s)", it is intended to mean the quantity of all X(s) present in the composition.

An additive can have several functions.

The composition according to the invention, comprises preferably more than one additive.

The total quantity of additive(s) is preferably of at least 0.1% by weight, more preferably of at least 0.3% by weight, even more preferably of at least 2% by weight based on the weight of the composition.

The total quantity of additive(s) is preferably of at most 30% by weight, more preferably of at most 25% by weight, even more preferably of at most 20% by weight based on the weight of the composition.

By "total quantity of additive(s)", it is intended to mean the quantity of all additive(s) present in the composition.

Advantageously, the composition according to the invention, further comprises a base oil other than 2-hexyl-1-decyl heptanoate and propylene glycol dicaprylate/dicaprate.

The American Petroleum Institute (API) has categorized base oils into five groups:
- Group I-III base oils are mineral oils refined from petroleum crude oil:
   - Group I base oils have a saturated hydrocarbon content of less than 90 % by weight, an aromatic hydrocarbon content of more than 1.7 % by weight, a sulphur content of more than 0.03 % by weight, and a viscosity index between 80 and 120;
   - Group II base oils have a saturated hydrocarbon content of more than 90 % by weight, an aromatic hydrocarbon content of less than 1.7 % by weight, a sulphur content of less than 0.03 % by weight, and a viscosity index between 80 and 120;
   - Group III base oils have a saturated hydrocarbon content of more than 90 % by weight, an aromatic hydrocarbon content of less than 1.7 % by weight, a sulphur content of less than 0.03 % by weight, and a viscosity index greater than 120;
   the percentages by weight being based on the weight of the base oil;
- Group IV base oils are synthetic oils, such as polyalphaolefins;
- Group V is for all other base oils not included in any of Groups I to IV.

The base oil may be chosen among Group I -V base oils, as long as it is neither 2-hexyl-1-decyl heptanoate nor propylene glycol dicaprylate/dicaprate.

The total quantity of 2-hexyl-1-decyl heptanoate and propylene glycol dicaprylate/dicaprate may vary from 10% to 99.9% by weight based on the weight of the composition.

By "total quantity of 2-hexyl-1-decyl heptanoate and propylene glycol dicaprylate/dicaprate", it is intended to mean the quantity of both 2-hexyl-1-decyl heptanoate and propylene glycol dicaprylate/dicaprate present in the composition.

Preferably, the total quantity of 2-hexyl-1-decyl heptanoate and propylene glycol dicaprylate/dicaprate represents at least 10%, more preferably at least 15%, even more preferably at least 20% by weight, such as at least 25% or 30% by weight based on the weight of the composition.

Preferably, the total quantity of 2-hexyl-1-decyl heptanoate and propylene glycol dicaprylate/dicaprate represents at most 99.9% by weight, more preferably at most 98% by weight based on the weight of the composition.

In a first embodiment, the total quantity of 2-hexyl-1-decyl heptanoate and propylene glycol dicaprylate/dicaprate represents less than 50% by weight based on the weight of the composition.

A preferred composition of the first embodiment, comprises a total quantity of 2-hexyl-1-decyl heptanoate and propylene glycol dicaprylate/dicaprate between 10 and 49.9% by weight; wherein the weight ratio 2-hexyl-1-decyl heptanoate / propylene glycol dicaprylate/dicaprate is of about 70/30; weight percentages being based on the weight of the composition.

In particular, the remaining 50.1-90% by weight are one or more additive(s) and/or a base oil other than 2-hexyl-1-decyl heptanoate and propylene glycol dicaprylate/dicaprate.

A particularly preferred composition of the first embodiment comprises or consists of:
- 10-49.9% by weight of:
   ∘ 2-hexyl-1-decyl heptanoate; and
   ∘ propylene glycol dicaprylate/dicaprate;
      wherein the weight ratio 2-hexyl-1-decyl heptanoate / propylene glycol dicaprylate/dicaprate is of about 70/30;
- 0.1-30% by weight of one or more additive(s);
- 50-89.9% by weight of a base oil other than 2-hexyl-1-decyl heptanoate and propylene glycol dicaprylate/dicaprate;
weight percentages being based on the weight of the composition.

In a second embodiment, the total quantity of 2-hexyl-1-decyl heptanoate and propylene glycol dicaprylate/dicaprate represents at least 50% by weight based on the weight of the composition.

A preferred composition of the second embodiment, comprises a total quantity of 2-hexyl-1-decyl heptanoate and propylene glycol dicaprylate/dicaprate between 50 and 99.9% by weight; wherein the weight ratio 2-hexyl-1-decyl heptanoate / propylene glycol dicaprylate/dicaprate is of about 70/30; weight percentages being based on the weight of the composition.

In particular, the remaining 0.1-50% by weight are one or more additive(s) and/or a base oil other than 2-hexyl-1-decyl heptanoate and propylene glycol dicaprylate/dicaprate.

A particularly preferred composition of the second embodiment comprises or consists of:
- 50-99.9% by weight of:
   ∘ 2-hexyl-1-decyl heptanoate; and
   ∘ propylene glycol dicaprylate/dicaprate;
      wherein the weight ratio 2-hexyl-1-decyl heptanoate / propylene glycol dicaprylate/dicaprate is of about 70/30;
- 0.1-30% by weight of one or more additive(s);
- 0-48% by weight of a base oil other than 2-hexyl-1-decyl heptanoate and propylene glycol dicaprylate/dicaprate;
weight percentages being based on the weight of the composition.

In a specific embodiment of this second embodiment, the composition according to the invention comprises or consists of:
- 70-99.9% by weight of:
   ∘ 2-hexyl-1-decyl heptanoate; and
   ∘ propylene glycol dicaprylate/dicaprate;
   wherein the weight ratio 2-hexyl-1-decyl heptanoate / propylene glycol dicaprylate/dicaprate is of about 70/30; and
- 0.1-30% by weight of one or more additive(s) used in the field of lubricants;
weight percentages being based on the weight of the composition.

The present invention also relates to a process for preparing the composition according to the invention, by mixing:
- 2-hexyl-1-decyl heptanoate;
- propylene glycol dicaprylate/dicaprate; and
- optionally one or more additive(s) and/or a base oil other than the 2-hexyl-1-decyl heptanoate and propylene glycol dicaprylate/dicaprate.

The components of the composition according to the invention can be added in any order.

Preferably, the mixing is performed at room temperature, such as between 20 and 40°C, in particular between 25 and 35°C.

The composition according to the invention is particularly suitable to be used in the field of lubricants.

The present invention also concerns an use of the composition according the invention, as a lubricant.

The lubricant is preferably a gear oil, a transmission fluid, or an hydraulic fluid.

In other words, the present invention concerns a method for lubricating one or more device(s), by bringing into contact the device(s) with the composition according to the invention.

The device(s) may be a gear or a transmission, in particular the device(s) is/are in an electric vehicle.

The present invention also concerns an use of the composition according the invention, as a liquid dielectric coolant.

Preferably, the liquid dielectric coolant is used as an electrical insulation and cooling medium for electrical device(s), such as transformers, servers, switches, rectifiers, cables, and capacitors.

The present invention concerns thus a method for lubricating and/or cooling one or more device(s), by bringing into contact the device(s) with the composition according to the invention.

In a particular embodiment, the composition according to the invention may be used to lubricate and to cool device(s) present in an electric vehicle.

Preferably, the device(s) is a gear, an electric motor, a transmission, a battery, and/or the power electronics of an electric vehicle.

The combination according to the invention having a pour point of -64°C, makes it possible to lower the pour point of a base oil other than the 2-hexyl-1-decyl heptanoate and propylene glycol dicaprylate/dicaprate, when mixed with said base oil.

The present invention also relates to an use of 2-hexyl-1-decyl heptanoate and propylene glycol dicaprylate/dicaprate to lower the pour point of a base oil other than the 2-hexyl-1-decyl heptanoate and propylene glycol dicaprylate/dicaprate; wherein the weight ratio 2-hexyl-1-decyl heptanoate / propylene glycol dicaprylate/dicaprate is of about 70/30.

In other words, the present invention also relates to a method for lowering the pour point of a base oil other than 2-hexyl-1-decyl heptanoate and propylene glycol dicaprylate/dicaprate, comprising mixing said base oil with 2-hexyl-1-decyl heptanoate and propylene glycol dicaprylate/dicaprate, wherein the weight ratio 2-hexyl-1-decyl heptanoate and propylene glycol dicaprylate/dicaprate is of about 70/30.

The 2-hexyl-1-decyl heptanoate, propylene glycol dicaprylate/dicaprate, and the base oil, are as described above, including preferential features and embodiments.

Preferably, the pour point of the base oil is higher than the pour point of the combination of 2-hexyl-1-decyl heptanoate and propylene glycol dicaprylate/dicaprate. In particular, the pour point of the base oil is of -60°C or higher.

The invention is further described in the following examples, given by way of illustration.

### Example 1: Combination according to the invention and comparative combinations

### 1.1. Chemicals used

- propylene glycol dicaprylate/dicaprate: Radia 7208 from Oleon;
- 2-hexyl-1-decyl heptanoate: was prepared by esterification of 2-hexyl-1-decanol (Isofol 16 from Sasol) with heptanoic acid (Oleris n-heptanoic acid from Arkema).

### 1.2. Preparation of the combination according to the invention

The combination C1 was prepared by mixing 30 wt% of propylene glycol dicaprylate/dicaprate with 70 wt% of 2-hexyl-1-decyl heptanoate at room temperature (25°C).

### 1.3. Preparation of comparative combinations

The comparative combination CC1 was prepared by mixing 20 wt% of propylene glycol dicaprylate/dicaprate with 80 wt% of 2-hexyl-1-decyl heptanoate at room temperature (25°C).

The comparative combination CC2 was prepared by mixing 40 wt% of propylene glycol dicaprylate/dicaprate with 60 wt% of 2-hexyl-1-decyl heptanoate at room temperature (25°C).

### Example 2: Pour point of the combinations

Pour points were measured for each ester and combination according to standard ASTM D97.

The pour point of 2-hexyl-1-decyl heptanoate was of -57°C.

The pour point of propylene glycol dicaprylate/dicaprate was of -41°C.

Each delta pour point was calculated relative to the lowest pour point among the esters contained in the corresponding combination. e.g. the pour point of 2-hexyl-1-decyl heptanoate (-57°C).

Results are gathered in Table 1.

**Table 1: Combinations and their pour point**

| | 2-hexyl-1-decyl heptanoate (w/w%) | Propylene glycol dicaprylate/dicaprate (w/w%) | Pour point (°C) | Delta pour point (°C) |
|---|---|---|---|---|
| C1 | 70 | 30 | -64 | -7 |
| CC1 | 80 | 20 | -49 | +8 |
| CC2 | 60 | 40 | -55 | +2 |

The combination C1 presents a pour point lower by -7°C than the lowest pour point among 2-hexyl-1-decyl heptanoate (-57°C) and propylene glycol dicaprylate/dicaprate (-41°C).

On the opposite, comparative combinations CC1 and CC2 with a weight ratio 2-hexyl-1-decyl heptanoate / propylene glycol dicaprylate/dicaprate out of the range, do not present a pour point lower than the lowest pour point of the esters contained in the comparative combination, e.g. the pour point of 2-hexyl-1-decyl heptanoate that is of -57°C. With -49°C for CC1 and -55°C for CC2, the pour points of the comparative combinations are comprised between the highest (-41°C) and the lowest (-57°C) pour point of the esters as usually expected.

## Claims

1. Combination consisting of:
- 2-hexyl-1-decyl heptanoate; and
- propylene glycol dicaprylate/dicaprate;
wherein the weight ratio 2-hexyl-1-decyl heptanoate / propylene glycol dicaprylate/dicaprate is of about 70/30.

2. Composition comprising:
- 2-hexyl-1-decyl heptanoate;
- propylene glycol dicaprylate/dicaprate; and
- at least an additive;
wherein the weight ratio 2-hexyl-1-decyl heptanoate / propylene glycol dicaprylate/dicaprate is of about 70/30.

3. Composition according to claim 2, further comprising a base oil other than 2-hexyl-1-decyl heptanoate and propylene glycol dicaprylate/dicaprate.

4. Process for preparing the composition according to claim 2 or 3, by mixing:
- 2-hexyl-1-decyl heptanoate;
- propylene glycol dicaprylate/dicaprate; and
- optionally one or more additive(s) and/or a base oil other than the 2-hexyl-1-decyl heptanoate and propylene glycol dicaprylate/dicaprate.

5. Use of the composition according to claim 2 or 3, as a lubricant.

6. Use of the composition according to claim 2 or 3, as a liquid dielectric coolant.

7. Use of 2-hexyl-1-decyl heptanoate and propylene glycol dicaprylate/dicaprate to lower the pour point of a base oil other than the 2-hexyl-1-decyl heptanoate and propylene glycol dicaprylate/dicaprate; wherein the weight ratio 2-hexyl-1-decyl heptanoate / propylene glycol dicaprylate/dicaprate is of about 70/30.
